# EUROPEAN PATENT APPLICATION

(11) **EP 4 115 802 A1**
(43) Date of publication of application: **11.01.2023**
(21) Application number: 21184406.3
(22) Date of filing: 08.07.2021
(51) Int. Cl.: A61B 5/097

(54) **DEVICE FOR COLLECTING EXHALED BREATH CONDENSATE AND THE USE OF SUCH DEVICE**

(30) Priority: 05.07.2021 PT 2021117324
(71) Applicant: INL - International Iberian Nanotechnology Laboratory, 4715-330 Braga (PT); Celoplas - Plásticos Para A Industria S.A., 4775-127 Grimancelos, Barcelos (PT); Digital Transformation CoLab, 4800-058 Guimarães (PT)
(72) Inventor: Yourievich Petrovykh, Dmitri, Braga (PT); Miguel da Cunha Oliveira, João, Guimarães (PT); Augusto Sousa Marques da Rocha, Luís, Braga (PT); de Oliveira Cortez, João, Vila Nova de Famalicão (PT); Alexandra Rebelo Cortez, Ana, Vila Nova de Famalicão (PT); Cunha Reis Rodrigues Sampaio, Marcos, Braga (PT)
(74) Representative: AWA Sweden AB

(57) **Abstract**

A device for collecting exhaled breath condensate (1000) is provided. The device comprises a condenser element (200) and a tubular member (300) with the tubular member fully or partly encircling the condenser element (200), thereby defining a longitudinally extending air passage (600) between an outer envelope surface (201) of the condenser element and an inner envelope surface (301) of the tubular member. A breath inlet end closure (500) comprising an inlet (501) is arranged at a lower end (202) of the device and in communication with the air passage. A condensate collection container (400) is arranged in an interface between a lower end of the condensation element and an upper open end of the breath inlet end closure. The condensate collection container comprises a mouth (402) closed by a check valve (401). The lower end of the condenser element is configured to selectively act on the check valve thereby opening a liquid passage allowing condensate to enter the condensate collection container. Also, the invention refs to the use of such device and to a condensate collection container as such.

## Description

### Technical field

The present invention refers to a device for collecting exhaled breath condensate, the use of such device and also a condensate collection container to be used in a device for collecting exhaled breath condensate.

### Technical background

It is well known in the art to collect condensate from a user's breath and use the collected condensate to examine a medical condition or actual conditions inside the user's respiratory tract. This is made by using a device for collecting exhaled breath condensate, often referred to as EBC. Non-limiting examples of parameters that may be examined are pH level in the condensate or presence of markers, for instance, of airway inflammation, such as hydrogen peroxide (H₂O₂), nitrogen oxides (NOx), and proteins. The collected condensate may be collected in a detachable container for further transport and examination.

It is known to rely on gravity to form a pool of condensate from which a sample for testing may be drawn. This may be made by guiding the user's exhaled breath to meet a cooler surface, onto which the humidity in the breath condenses and forms droplets, and where the droplets fall by gravity into a compartment from which the condensate may be extracted for further examination.

Since these kind of devices may be used both at home by the user herself or in medical clinics while the user is supervised by trained personnel, it is essential that the devices are easy to use, and that the collected condensate is protected throughout the whole chain from collection to examination, and not the least that the person handling the device does not come in contact with the condensate. It is also essential that the collected condensate is easily accessible in the clinical laboratory.

### Summary

One objective of the present invention is to provide a simple self-contained and portable device for efficient collection, storage and transport of condensate that is derived from a user's exhaled breath.

A further objective is to provide a device in which a condensate sample collected from a user's exhaled breath may be collected and be subjected to various laboratory tests without the risk of contamination by exposure to external influences and also without exposing the person handling the device.

Yet another objective is to provide a device which may be made available for use in a medical clinic by medically trained personnel or by the patient herself at home.

A further objective of the invention is to provide a device in which condensate may be collected, stored and transported in a single self-contained unit.

Yet another purpose is to provide a condensate collection container that may be used as a disposable single-use container in a breath condensate collection device.

These and other objects are solved by a device for collecting exhaled breath condensate, said device comprising a condenser element and a tubular member which fully or partly encircles the condenser element and which defines a longitudinally extending air passage between an outer envelope surface of the condenser element and an inner envelope surface of the tubular member;
a breath inlet end closure comprising an inlet, said breath inlet end closure being arranged at a lower end of the device and in communication with the air passage; and
a condensate collection container being arranged in an interface between a lower end of the condensation element and an upper open end of the breath inlet end closure, the condensate collection container comprising a mouth closed by a check valve; and wherein
the lower end of the condenser element is configured to selectively act on the check valve thereby opening a liquid passage allowing condensate to enter the condensate collection container.

By the present invention, a device for collecting exhaled breath condensate is provided which has a simple design and which is easy to use no matter if the device is used by a patient on her own or by trained personnel at a medical clinic. This is of importance in terms of hygiene and personal safety, since body fluids are handled.

By the present invention, there is no risk that the person handling the device, or who disassembles and/or assembles the device, will come in contact with the collected condensate. Rather, the collected condensate will be safely collected in and contained in the condensate collection container and will be protected from escaping by the check valve. Further, the check valve ensures that there is no open surface of the collected condensate. Thereby evaporation of the collected condensate is prevented. The check valve is selectively openable, i.e. it will only be open if actively acted upon. This could be during a stage when the condensate collection container is mounted in the device and thereby is arranged in engagement with the lower end of the condenser element, whereby the lower end thereof will activate the check valve to open. It could also be made during the clinical examination of the collected condensate. During the clinical examination, the check valve may either be selectively acted upon by e.g. a pipette, or the check valve is deliberately removed. In any other situation, the check valve and the condensate collection container will together be a unitary body forming a closed compartment that prevents the collected condensate from escaping or evaporation. Further, it protects the user from coming in contact with the collected condensate. Thus, the present invention allows a facilitated and safe handling or transport of the condensate collection container.

The condensate collection container and its check valve may be provided as a disposable item, whereas the rest of the device may be provided for reuse. It is preferred that the inlet is provided with a disposable mouthpiece.

The condenser element may be a hollow compartment configured to contain a refrigerant such as water or ice cubes or be a solid body which is configured to be pre-cooled and act as a removable cooling cartridge. The condenser element may also be configured to contain a cooling thermoelectric element.

Further, while the cooling element during use of the device has a temperature that is lower than room temperature, the tubular member will during use of the device remain close to room temperature. Since the cooling element is separated from the tubular member, the user's hands will not warm the envelope surface. In fact, the air passage that is formed between the cooling element and the tubular member will act as a thermal insulation.

Accordingly, and in summary, the present invention provides a device for collecting exhaled breath condensate which is easy to use no matter the degree of experience, which allows a high level of hygiene, reducing the risk of contamination, both of the collected condensate and of the person handling the condensate collection container.

The condenser element may have an outer cone-shaped envelope surface.

The cone-shaped envelope surface contributes to the guiding of droplets by gravity in the longitudinal direction towards the condensate collection container. This allows an improved overall efficiency of the device and hence a reduced number of breaths which are required to collect a sufficient volume of condensate from the breath. The collected volume should be sufficiently large to allow a proper examination.

The outer cone-shaped envelope surface may have a convex extension as seen along the longitudinal extension of the condenser element. The convex extension allows an enhanced contact surface with the exhaled air as compared to a straight conical envelope surface. This allows an improved overall efficiency of the device and hence a reduced number of breaths which are required to collect a sufficient volume of condensate from the breath.

The outer envelope surface of the condenser element may be provided with a coating or a texture. The coating or texture is configured to reduce resistance, whereby movement of the condensate droplets on the surface of the cooling element may be facilitated. Since the device relies on gravity to form a condensate pool, the coating or texture may contribute to overcome surface tension and hence to overcome water's naturally tendency to stick to the walls of the condenser element. As a non-limiting example of a coating, polytetrafluoroethylene may be used.

The condensate collection container may be removably attached to the device for collecting exhaled breath condensate. This allows the condensate collection container to be detached and handled as a separate member that can be transported to the laboratory for analysis. Thus, the condensate collection container may be handled as a disposable item.

The condenser element may be removably received in the breath condensate collection device. This allows the condenser element to be cooled separately from the device and be arranged in the device shortly before use.

A lower end portion of the tubular member may sealingly connect to the upper open end of the breath inlet end closure. By the sealing connection, any air from the user's exhalation will be prevented from escaping the air passage before having passed along the outer envelope surface of the condenser element. This increases the overall efficiency of the device and hence the number of breaths which are required to collect a sufficient volume of condensate.

The check valve may be configured to selectively close the mouth of the condensate collection container.

The check valve is configured to be operated by the condenser element acting thereon. When the condenser element is mounted on the device, the lower end of the condenser element will act on the check valve, thereby setting the same to an open position, in which a liquid passage is formed that allows condensate to enter the condensate collection container. Correspondingly, when the condenser element is removed from the device, the check valve will automatically close and thereby close-off the liquid passage. Thereby any collected condensate is prevented from leaving the condensate collection container and also foreign matter is prevented from entering the condensate collection container during handling.

The check valve may be removably arranged to the condensate collection container. Alternatively, the check valve may be fixedly arranged to the condensate collection container.

In the event the check valve is removably arranged to the condensate collection container, the check valve may be actively removed in the laboratory to allow access to the collected condensate inside the condensate collection container.

Alternatively, in the event of the check valve that is fixedly arranged to the condensate collection container, access to the collected condensate may be made via the check valve by using a pipette or the like. No matter how the check valve is arranged to the condensate collection container, it will protect the collected condensate from the moment the container is dismounted from the device. This may be seen as a hygienic safety measure but also a quality aspect.

The device may further comprise a lid, said lid being configured to be removably arranged to an upper end portion of the tubular member or to an upper end portion of the condenser element.

The lid may ensure that the condenser element when received in the tubular member acts on the check valve thereby setting the same to an open position and thereby opening a liquid passage which allows condensate to enter the condensate collection container. This may be ensured by the lid in condition when being correctly mounted to the device being configured to push the condenser element in the longitudinal direction.

The lid and any filter may also contribute to preventing any foreign matter to enter the air passage and or the condenser element in the event the latter is hollow.

The lid may be air permeable.

The permeability to air may be provided for by the lid being provided with one or more through openings, perforations or even with a flexible membrane. It is preferred that any openings or perforations are provided with a filter. The filter material may be of the same type that are used in face masks or surgical masks. The filter will prevent contagious aerosol from escaping. The filter may be made from non-woven fabrics. The non-woven fabrics may be made from plastics like polypropylene, which can be of 20 or 25 grams per square meter in density. The filter may alternately be made of polystyrene, polycarbonate, polyethylene, or polyester. By the filtered outlet openings, nearby persons are protected from the breath during the collection of exhaled breath condensate.

In the event of a flexible membrane, the membrane should be flexible to allow the inherent force in the user's exhalation to open said membrane to thereby allow the air to be released from the air passage. As the exhalation stops, the flexible membrane will sealingly close off the air passage. Thus, the flexible membrane acts as a check valve. The user will thereby be encouraged by the design to breath in via the nose, since breathing in via the mouth is blocked. For the specific purpose of testing for e.g. COVID infection, this is advantageous, as in the process of breathing in through the nose and breathing out through the mouth, the most relevant parts of the upper respiratory tract are sampled.

The tubular member may in an upper end portion thereof comprise at least one air outlet. Thereby air from the user's exhalation may be released from the air passage. It is preferred that said at least one air outlet is provided with a filter. The filter material may be of the same type that are used in face masks or surgical masks. The filter will prevent contagious aerosol from escaping.

The condenser element may comprise a hollow a compartment configured to contain a refrigerant or be a solid body.

In event the condenser body comprises a hollow compartment, this compartment may be configured to be filled with a cooled refrigerant such as water, a slurry with water and ice or ice cubes. The skilled person realizes that the hollow compartment with remained function may be filled with other types of refrigerants and even a thermoelectric cooling element. Alternatively, the condenser element may be a solid body which is configured to be pre-cooled and act as a removable cooling cartridge. Yet another possibility is to provide the condenser element in the form of a reusable cooling cartridge comprising a closed hollow element that is filled with a refrigerant.

The condenser element is configured to be cooled before use to provide a relative temperature difference between the cooling element and the other parts of the device and especially the tubular member and the end closure, where the temperature of the cooling element is lower than the other parts of the device.

According to another aspect, the invention refers to the use of a device according to any of claims 1-13, for collection of breath condensate. The device and its advantages has been thoroughly discussed above and those features and advantages are equally applicable to the use of such device.

According to yet another aspect, a condensate collection container to be used in a device for collecting exhaled breath condensate is provided. The condensate collection container comprises a compartment having a mouth covered by a check-valve; wherein said check-valve is configured to be set between: a closed position in which the compartment is closed; and an open position in which a liquid passage is formed into the compartment, thereby allowing a condensate to enter said compartment.

The condensate collection container as such and its advantages have been thoroughly discussed above in relation to the breath condensate collection device. Those features and advantages are equally applicable to the use of the condensate collection device as a stand-alone unit and especially as a disposable single-use container to be used in a breath condensate collection device.

Especially, the check valve is selectively openable, i.e. it will only be open if actively acted upon. This could be during a stage when the condensate collection container is mounted in a breath condensate collection device and thereby is arranged in engagement with the lower end of the condenser element, whereby the lower end thereof will activate the check valve to open. It could also be made during clinical examination of the collected condensate. During the clinical examination, the check valve may either be selectively acted upon by e.g. a pipette, or the check valve is deliberately removed. In any other situation the check valve and the condensate collection container will together be unitary body forming a closed compartment which prevents the collected liquid from escaping or coming in contact with the user. Thus, the present invention allows a facilitated and safe handling or transport of the condensate collection container

Further objects and advantages of the present invention will be obvious to a person skilled in the art reading the detailed description given below describing different embodiments.

### Brief description of the drawings

The invention will be described in detail with reference to the schematic drawings.
Fig. 1 is a perspective view of one embodiment of the breath condensate collection device.
Fig. 2 is an exploded view of the breath condensate collection device.
Fig. 3 is a view of the condensate element
Fig. 4 is a view of the lid.
Fig. 5 is a view of the condensate collection container with the check valve.
Fig. 6 is a view of the breath inlet end closure.
Fig. 7 is a view of the tubular member.
Fig. 8 is a perspective view of the device for collecting exhaled breath condensate illustrating its use.

### Detailed description

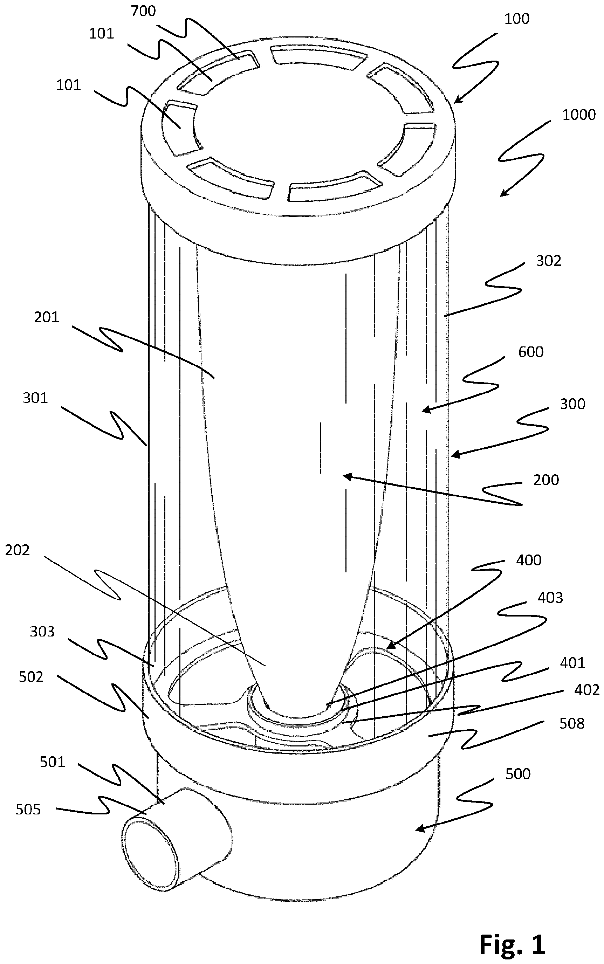

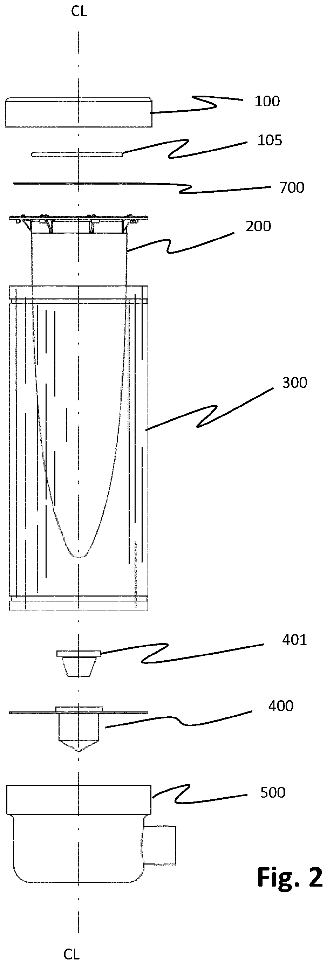

Now turning to Figs. 1 and 2, the overall design of one embodiment of the device for collecting exhaled breath condensate 1000 is disclosed. The device for collecting exhaled breath condensate 1000 will in the following, unless nothing else is given, be referred to as *"the device".*

The device 1000 has a longitudinal extension as seen along a longitudinal centreline CL.

The device 1000 comprises, starting from the top, a lid 100, a condenser element 200, a tubular member 300, a condensate collection container 400 with a check valve 401, and a breath inlet end closure 500. The tubular member 300 is arranged to fully or partly encircle the condensate element 200, thereby defining a longitudinally extending air passage 600 between an outer envelope surface 201 of the condenser element 200 and an inner envelope surface 301 of the tubular member 300. A lower end of the tubular member 300 is connected to the breath inlet end closure 500 which comprises an inlet 501. The inlet 501 is arranged in fluid communication with the air passage 600. The condensate collection container 400 is arranged in an interface between a lower end 202 of the condenser element 200 and an upper open end 502 of the breath inlet end closure 500. The condensate collection container 400 comprises a mouth 402 which is closed by the check valve 401. A lower end 201 of the condenser element 200 is configured to selectively act on the check valve 402 to thereby open a liquid passage 403 into the condensate collection container 400.

The underlying principle of the device 1000 may shortly be described as follows: The condenser element 200 is configured to be cooled before use of the device 1000 to provide it with a temperature that is lower than the room temperature. Thus, there should be a relative temperature difference between the cooling element and the room temperature. The skilled person also realises that the condenser element 200 may be cooled during use, e.g. when ice or ice water is used as a refrigerant. As the user breathes into the inlet 501, the breath will enter the breath inlet end closure 500 and travel along the air passage 600 which is formed between the outer envelope surface 201 of the condenser element 200 and the inner envelope surface 301 of the tubular member 300. The humidity in the warm breath will condensate on the outer envelope surface 201 of the relatively colder condenser element 200. The thus formed droplets will travel by gravity in the opposite direction and enter the condensate collection container 400 via the liquid passage 403. The breath will escape the air passage 600 in the opposite direction via one or more filtered openings 101 in the lid 100.

In the following the individual components of the device 1000 will be described.

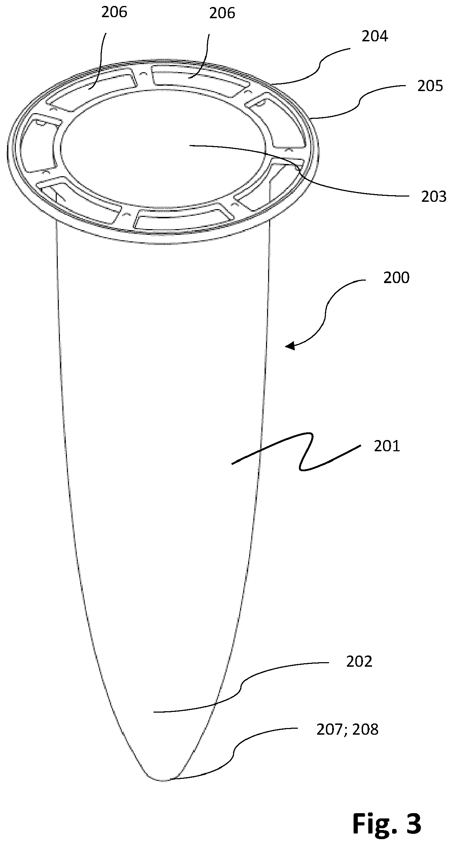

Now turning to Fig. 3, one embodiment of the condenser element 200 is disclosed. The condenser element 200 is in the disclosed embodiment formed as a hollow member having a compartment 203 which is configured to contain a refrigerant or a thermoelectric cooling element. The refrigerant may as non-limiting examples be cold water, ice cubes or a slurry of ice and water. The skilled person realizes that the same functionality may be achieved by the condenser element 200 instead being formed as a solid or hollow body which is configured to be pre-cooled and act as a removable cooling cartridge.

The condenser element 200 is configured to be cooled before use to provide a relative temperature difference between the cooling element 200 and the other parts of the device and especially the tubular member 300 and the end closure 500.

The condenser element 200 has a longitudinal extension along the longitudinal centreline CL. The condenser element 200 has a cone-shaped outer envelope surface 201. The cone-shaped outer envelope surface 201 contributes to the guiding of droplets by gravity in the longitudinal direction towards the condensate collection container 400. This allows an improved overall efficiency of the device 1000 and hence a reduced number of breaths which are required to collect a sufficient volume of condensate from the breath.

The outer cone-shaped envelope surface 201 is disclosed as having a convex extension as seen along the longitudinal extension of the condenser element 200. The convex extension allows an increased contact surface with the exhaled air as compared to a straight conical envelope surface. This allows an improved overall efficiency of the device and hence a reduced number of breaths which are required to collect a sufficient volume of condensate from the breath in a short period of time. The skilled person realizes that the outer cone-shaped envelope surface 201 with remained functionality may have a straight extension as seen along the longitudinal extension of the condenser element 200.

The outer envelope surface 201 may optionally be provided with a coating or a texture (not disclosed) configured to reduce resistance for the droplets to move along the surface 201.

An upper end 204 of the condenser element 200 comprises a radially extending flange 205. The flange 205 is provided with a plurality of through-going openings 206. The flange 205 is used to form a radially extending support for the condenser element 200 in a condition when the condenser element 200 is mounted to the device 1000 via the tubular member 300.

The lower end 202 of the condenser element 200 comprises an actuator portion 207 which is configured to engage the check valve 402 and set the same to an open position. The actuator portion 207 is in the disclosed embodiment formed by the apex 208 of the cone-shaped envelope surface 201. The apex 208 is provided with a rounded tip. Alternatively, the tip may be truncated. It is to be understood that other designs also may be used with retained function. The actuator portion 207 must, by way of example, not be integrally formed with condenser element 200, but may instead be provided as a separate member to be mounted thereto. No matter the design, it is of relevance that the actuator portion 207 is complementary to the check valve 402 to allow actuation thereof in a condition when the condenser element 200 is properly mounted to the device 1000.

The condenser element 200, no matter if this is designed as a hollow container or as a solid body, may be formed by a plastic material, a composite material or a metallic material.

The condenser element 200 is preferably configured to be removably received in tubular member 300. This allows the condenser element 200 to be cooled separately from the device 1000 and be arranged in the device shortly before use.

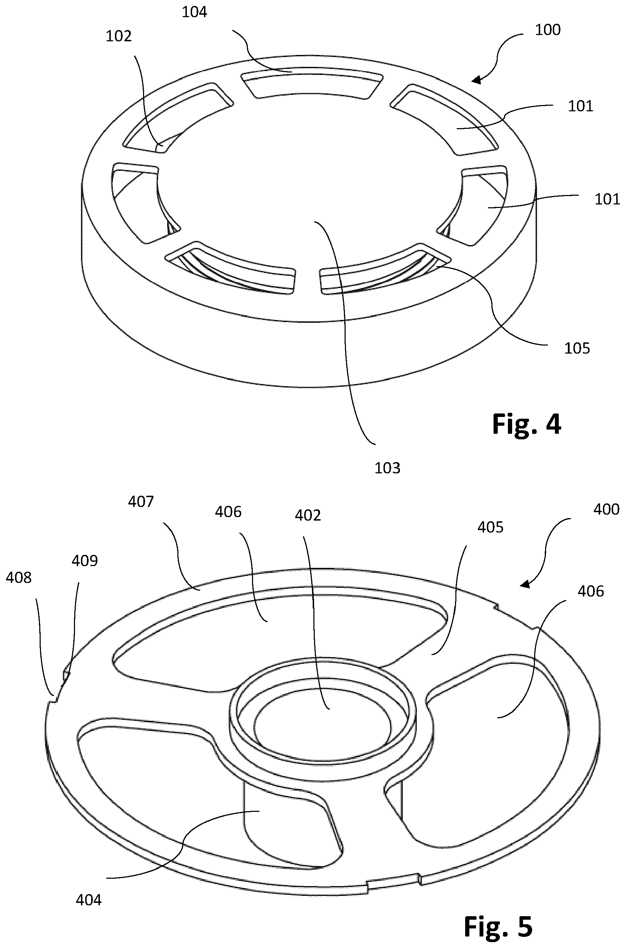

Now turning to Figs. 1 and 4, one embodiment of the lid 100 is disclosed. The lid 100 is configured to be removably arranged to an upper end portion 302 of the tubular member 300 to thereby close the open end thereof. The lid may as non-limiting examples be provided with a locking member 102, e.g. a thread, a snap fit or the like, which is configured to engage a complementary locking member 304 in the upper end 302 of the tubular member 300.

A major top surface 103 of the lid 100 is provided with a plurality of through-going openings 101. The through-going openings 101 may optionally be provided with an air-permeable filter 700. The openings 101 and the filter 700 may be replaced by the major surface 103 instead being provided with perforations. No matter configuration, the openings or perforations are used to allow the users breath which is configured to travel along the air passage 600 to leave the device 1000. The air-resistance should be minimal. The filter material may be of the same type that is used in face masks.

The inner major surface 104 of the lid 100 is configured to abut the condenser element 200 when received in the tubular member 300. The abutment is in the disclosed embodiment provided for by the inner major surface 104 of the lid 100 contacting the upper surface of the radially extending flange 204 of the condenser element 200. The lid 100 is thereby configured to push the condenser element 200 in the longitudinal direction. The opposite free end of the condenser element 200 will thereby act on the check valve 401 and set the same to an open position and thereby open the liquid passage 403 which allows condensate to enter the condensate collection container 400. Accordingly, the liquid passage 403 is only formed when the condenser element 200 and the lid 100 are correctly mounted to the tubular member 300.

Further, the inner major surface 104 of the lid 100 comprises a lip 105 configured to sealingly close-off the compartment 203 of the condenser element 200. Thereby, the refrigerant is prevented from leaving the condenser element 200.

The lid 100 contributes to preventing any foreign matter, such as dust, to enter the air passage 600 that is formed between the inner wall 301 of the tubular member 300 and the outer envelope surface 201 of the condenser element 200.

Now turning to Figs 1 and. 5, one embodiment of the condensate collection container 400 is disclosed. The condensate collection container 400 is preferably configured to be removably attached in the device 1000. This allows the condensate collection container 400 to be detached and handled as a separate container which can be shipped to the laboratory for analysis. The condensate collection container 400 may be provided as a disposable item and which is provided for single use.

The condensate collection container 400 comprises a longitudinally extending compartment 404 which is aligned with the longitudinal centreline CL of the condenser element 200. The compartment 404 has a mouth 402 configured to face the condenser element 200.

The condensate collection container 400 further comprises a radially extending scaffold 405. The scaffold 405 is provided with at least one through-going opening 406. The at least one opening 406 allows the breath to enter the air passage 600 with a minimal obstruction.

The scaffold 405 is configured to be supported by the upper end of the breath inlet end closure 500 and be clamped between the lower end of the tubular member 300 and the breath inlet end closure 500 when the condensate collection container 400 is mounted to the device 1000.

An outer, circumferentially extending edge 407 of the scaffold 405 is in the disclosed embodiment provided with guiding members 408. The guiding members are disclosed as indents 409. The indents 409 are configured to allow the scaffold to linearly pass locking members 507 of the breath inlet end closure 500 when mounting the scaffold 405 in the breath inlet end closure 500.

An upper edge portion of the mouth 402 of the condensate collection container 400 supports a check valve 401, see Fig. 1. The check valve 401 is configured to selectively close/open the mouth 402. More precisely, the check valve 401 is configured to be set to an open position when the condenser element 200 acts on the check valve 401, thereby forming a liquid passage 403 into the condensate collection container 400. Correspondingly, the check valve 401 is configured to be set to a closed position when the condenser element 200 is removed, thereby closing-off the liquid passage 403.

The check-valve may by way of example be of the type comprising a biased ball, or a biased slider which is configured to selectively open/close one or more openings. The skilled person is well familiar with check valves and their function.

The check valve 401 may be removably arranged to the condensate collection container 400. Alternatively, the check valve 401 may be fixedly arranged to the condensate collection container 400. In the event the check valve 401 is removably arranged to the condensate collection container 400, the check valve 401 may be configured to be removed in the laboratory to allow access to the collected condensate inside the condensate collection container 400.

Alternatively, in the event of the check valve 401 is fixedly arranged to the condensate collection container 400, access to the collected condensate may be made via the check valve 401 by using e.g. a pipette.

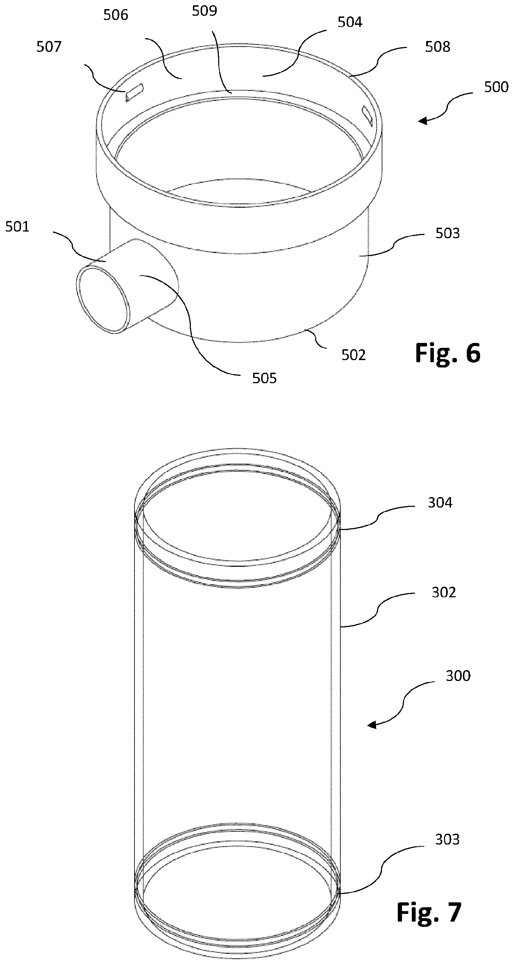

Now turning to Fig. 6, one embodiment of the breath inlet end closure 500 is disclosed. The breath inlet end closure 500 has a cup-shaped configuration with a closed bottom wall 502 and a circumferentially extending side wall portion 503. The side wall portion 503 delimits an open mouth 504 opposite the bottom wall 502.

The side wall portion 503 comprises the inlet 501. The inlet 501 is formed as a radially extending tube 505. When using the device 1000, the user is intended to exhale directly or indirectly via said tube 505. The exhalation may be made directly via the tube 505 or via a breathing mouthpiece (not disclosed) to be connected to the free end of the tube 505.

An inner wall portion 506 of the inner side wall portion 503 comprises a shoulder 509 configured to form a support for the scaffold 405 of the condensate collection container 400.

Further, the inner wall portion 506 of the side wall portion 503 comprises a locking member 507 in the form of a threaded portion. The locking member 507 is configured to lockingly engage a groove in the lower end portion 303 of the tubular member 300. Also, the locking member 507 prevents the condensate collection container 400 from accidentally being removed from the breath inlet end closure 500.

Now turning to Figs. 1, 2 and 7, the tubular member 300 is discussed. The tubular member is configured to extend between the lid 100 and the breath inlet end closure 500 to thereby form an outer longitudinally extending side wall of the device 1000.

A lower end portion 303 of the tubular member 300 is configured to sealingly and releasably connect to an upper open end 508 of the breath inlet end closure 500.

Thus, during assembly of the device 1000, the condensate collection container 400 is configured to be arranged across the opening of the breath inlet end closure 500 by the scaffold 405 being supported by the shoulder 509. Then the tubular member 300 is mounted to the breath inlet end closure 500 by arranging the lower end of the tubular member 300 to lockingly engage the locking member 507 of the breath inlet end closure 500. The engagement should preferably be sealed. Thereby air from the user's exhalation will be prevented from escaping the air passage 600 before having passed along the outer envelope surface 201 of the condenser element 200. The interface between the lower end portion 303 and the upper open end 508 of the breath inlet end closure 500 may be provided with a non-disclosed sealing member, such as an O-ring. This increases the overall efficiency of the device 1000 and hence the number of breaths which are required to collect a sufficient amount of condensate.

The upper end portion 302 of the tubular member 300 is provided with a locking member 304 in the form of a groove which is configured to sealingly and releasably connect with the locking member 102 of the lid 100.

The tubular member 300 may be formed by any type of material. It is however advantageously if the tubular member is formed of a transparent/translucent material. This allows to ensure by a visual inspection that the condenser element 200 is correctly mounted, i.e. that the lower end 202 thereof engages the check valve 401, and also that a condensate is properly formed during use of the device 1000. If no condensate is formed, it is likely that the condenser element 200 is not cool enough in relation to the breath of the user.

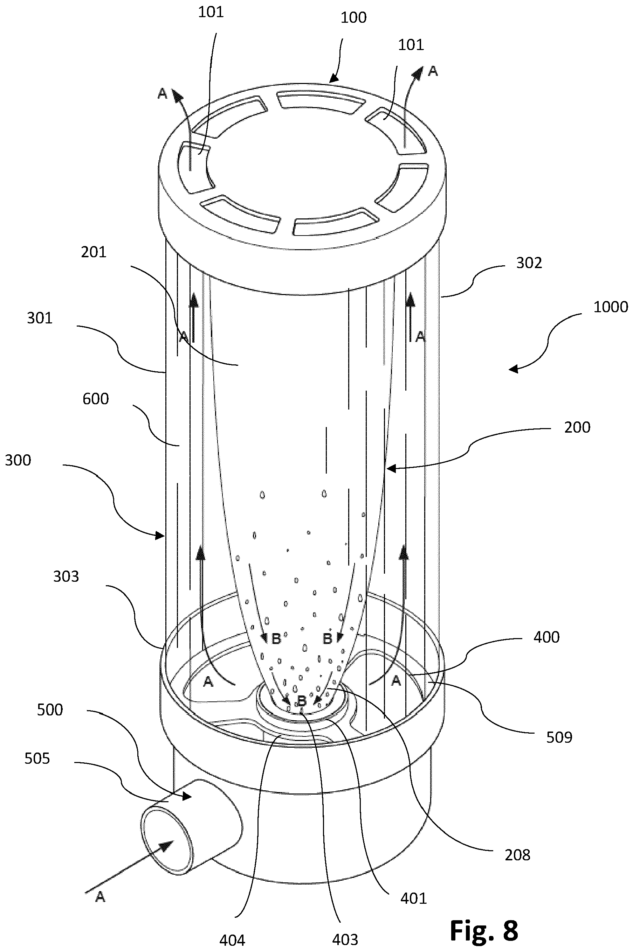

Now referring to Fig. 8, the use of the device 1000 according to the invention will be discussed. As given above, the purpose of the device 1000 is to collect breath condensate. The condensate may be used to examine a medical condition or actual conditions inside the user's respiratory tract. Non-limiting examples of parameters that may be examined are pH level in the condensate or presence of markers, for instance, of airway inflammation, such as hydrogen peroxide (H₂O₂), nitrogen oxides (NOx), and proteins. Also, presence of virus and bacteria may be examined.

As an initial step, the condensate collection container 400 is arranged in the breath inlet end closure 500 by the scaffold 405 being supported by the shoulder 509. Then, the lower end 303 of the tubular member 300 is sealingly engaged with the breath inlet end closure 500.

Further, the condensing element 200 is cooled down to a temperature well below the ambient temperature and well below the temperature of the user's breath. In the event the condensing element 200 has a hollow interior this may be made by filling the same with a refrigerant, such as ice, cooled water or a slurry of ice and water. Alternatively, the condensing element 200 may be a thermoelectric element. In the event the condenser element instead is a solid or hollow body acting as a cooling cartridge, it may be taken out from the freezer.

The cooled condenser element 200 is inserted into the tubular member 300 so that the apex 208 of the condenser element 200 abuts the check valve 401 and thereby sets the same to an open position to thereby form a liquid passage 403 into the container 404 of the condensate collection container 400. As a final step, the lid 100 is mounted to the upper end of the tubular member. The device is now ready to be used.

The user puts her lips directly or indirectly to the inlet tube 505 and exhales into the device 1000. The breath, see arrow A, will enter the breath inlet end closure 500 and then be guided in the longitudinal direction along the air passage 600 that is formed between the inner wall of the tubular element 301 and the exterior envelope surface 201 of the condenser element 200. As the breath comes into contact with the relatively cooler exterior envelope surface 201 of the condenser element 200, the humidity in the breath will condense and form droplets on the exterior envelope surface 201 of the condenser element 200. The thus formed droplets will follow, by gravity, the exterior surface envelope surface 201, see arrow B, and enter the container 404 of the condensate collection container 400 via the liquid passage 403. The air from the breath will leave the air passage 600 via the one more through-going openings 101 in the lid 100.

The breathing is continued until a sufficient amount of condensate has been pooled in the condensate collection container. The condensate collection container 400 may then be removed for further handling. The removal is made by releasing the engagement between the breath inlet end closure 500 and the lower end of the 303 of the tubular member 300 and then removing the condensate collection container 400. Further, the condenser element 200 may be removed by releasing the engagement between the lid 100 and the upper end 302 of the tubular member 300.

The check valve 401 will close-off the liquid passage 403 the very same time as the engagement between the condenser element 200 and the check valve 401 is released. Thus, the condensate collection container 400 may in its removed condition be handled as a closed container until clinical examination of the contents.

Accordingly, by the present invention, a device for collecting exhaled breath condensate is provided which has a simple design and which is easy to use, no matter if the device 1000 is used by a patient on her own or by trained personnel at a medical clinic. This is of importance in terms of user friendliness, hygiene and personal safety since body fluids are handled. There is no risk that the person using the device 1000, or which disassembles and/or assembles the device 1000 will come in contact with the collected condensate. Rather, the collected condensate will be safely collected in and contained in the condensate collection container 400 and will be protected from escaping by the check valve 401 and also prevent the user from coming in contact with the condensate.

The check valve 401 is selectively openable, i.e. it will only be open if actively acted upon. This could be during a stage when the condensate collection container 400 is mounted in the device 1000 and thereby is arranged in engagement with the lower end 202 of the condenser element 200 whereby the lower end thereof 202 will activate the check valve 401 to open the same. It could also be made during clinical examination of the collected condensate. During the clinical examination, the check valve 401 may either be selectively acted upon by e.g. a pipette., or the check valve 401 is deliberately removed. In any other situation the check valve 401 and the condensate collection container 400 will together be a unitary body forming a closed compartment which prevents the collected liquid from escaping or coming in contact with the user. Thus, the present invention allows a facilitated and safe handling or transport of the condensate collection container 400.

The condensate collection container 400 and its check valve 401 may be provided as a disposable item, whereas the rest of the device 1000 may be provided for reuse. It is preferred that the inlet tube 505 is provided with a disposable mouthpiece.

Accordingly, and in summary, the present invention provides a device for collecting exhaled breath condensate which is easy to use no matter degree of experience, which allows a high degree of hygiene and which reduces the risk of contamination, both of the collected condensate and of the person handling the condensate collection container.

The lid 100 has been disclosed as being removably arranged to an upper end portion of the tubular member 300. The skilled person realizes that the lid 100 with remained function may be designed to instead be removably arranged to an upper end portion of the condenser element 200.

The interface between the lid 100 and the condenser element 200 may in a non-disclosed embodiment be provided with a flexible membrane. The flexible membrane should be flexible enough to allow the inherent force in the user's exhalation to open said membrane to thereby allow the air to be released from the air passage. As the exhalation stops, the flexible membrane will sealingly close off the air passage. Thus, the flexible membrane acts as a check valve which closes off the air passage when the device is not actively used.

The tubular member 300 may in a non-disclosed embodiment, comprise at least one air outlet in an upper end portion thereof. Thereby air from the user's exhalation may be released from the air passage 600. Such at least one air outlet may replace the one or more through-going openings 101 in the lid 100.

The skilled person realizes that the device 1000 is not limited to be used with water-based refrigerants but that also other refrigerants may be used with remained function.

## Claims

1. A device (1000) for collecting exhaled breath condensate, said device comprising a condenser element (200) and a tubular member (300) with the tubular member (300) fully or partly encircling the condenser element (200), thereby defining a longitudinally extending air passage (600) between an outer envelope surface (201) of the condenser element (200) and an inner envelope surface (301) of the tubular member (300);
a breath inlet end closure (500) comprising an inlet (501), said breath inlet end closure (500) being arranged at a lower end (202) of the device (1000) and in communication with the air passage (600); and
a condensate collection container (400) being arranged in an interface between a lower end of the condensation element (200) and an upper open end of the breath inlet end closure (500), the condensate collection container (400) comprising a mouth (402) closed by a check valve (401); and wherein
the lower end (202) of the condenser element (200) is configured to selectively act on the check valve (401) thereby opening a liquid passage (403) allowing condensate to enter the condensate collection container (400).

2. The device according to claim 1, wherein the condenser element (200) has an outer cone-shaped envelope surface (201).

3. The device according to claim 2, wherein the outer cone-shaped envelope surface (201) of the condenser element (200) has a convex extension as seen along the longitudinal extension.

4. The device according to any of the preceding claims, wherein the outer envelope surface (201) of the condenser element (200) is provided with a coating or a texture configured to reduce resistance, thereby facilitating movement of condensate droplets on the surface of the cooling element.

5. The device according to any of the preceding claims, wherein the condensate collection container (400) is removably attached to the device for collecting exhaled breath condensate (1000).

6. The device according to any of the preceding claims, wherein the condenser element (200) is removably received in the device for collecting exhaled breath condensate (1000).

7. The device according to any of the preceding claims, wherein a lower end portion (302) of the tubular member (300) sealingly connects to the upper open end (508) of the breath inlet end closure (500).

8. The device according to any of the preceding claims, wherein the check valve (401) is configured to selectively close the mouth (402) of the condensate collection container (400).

9. The device according to any of the preceding claims, wherein the check valve (401) is removably arranged to the condensate collection container (400), or wherein the check valve (401) is fixedly arranged to the condensate collection container (400).

10. The device according to any of the preceding claims, wherein the device for collecting exhaled breath condensate (1000) further comprises a lid (100), said lid (100) being configured to be removably arranged to an upper end portion (301) of the tubular member (300) or to an upper end portion of the condenser element (200).

11. The device according to claim 10, wherein the lid (100) is air permeable.

12. The device according to any of claims 1-11, wherein the tubular member (300) in an upper end portion (302) thereof comprises at least one air outlet.

13. The device according to any of the preceding claims, wherein the condenser element (200) comprises a hollow compartment configured to contain a refrigerant or wherein the condenser element (200) is a solid body.

14. Use of a device according to any of claims 1-13, for collection of breath condensate.

15. A condensate collection container (400) to be used in a device for collecting exhaled breath condensate, said condensate collection container (400) comprising compartment (404) having a mouth (402) covered by a check-valve (401);
wherein said check-valve (401) is configured to be set between:
a closed position in which the compartment (404) is closed; and
an open position in which a liquid passage (403) is formed into the compartment (404), thereby allowing a condensate to enter said compartment (404).
